# EUROPÄISCHE PATENTANMELDUNG

(11) **EP 4 424 343 A1**
(43) Veröffentlichungstag der Anmeldung: **04.09.2024**
(21) Anmeldenummer: 23159889.7
(22) Anmeldetag: 03.03.2023
(51) Int. Cl.: A61M 5/168

(54) **INFUSIONSPUMPE MIT BEDIENEINHEIT**

(71) Anmelder: B. Braun Melsungen AG, 34212 Melsungen (DE)
(72) Erfinder: Angersbach, Klaus, 34326 Morschen (DE); Buerger, Maria, 34323 Malsfeld (DE); Erlen, Christoph, 34132 Kassel (DE); Höllerich, Mike, 50968 Köln (DE); Moeller, Sebastian, 37235 Hessisch Lichtenau (DE); Hupe, Jella Maria, 34590 Wabern (DE)
(74) Vertreter: Tergau & Walkenhorst Intellectual Property GmbH

(57) **Zusammenfassung**

Infusionspumpe (1) mit einem Gehäuse (2), einer Bedieneinheit (24), einer Antriebseinheit (18) zur Förderung einer Infusionslösung und einer Steuereinheit (12), die für Target Controlled Infusion von Infusionslösung durch einen Patientenanschluss an einen Patienten zur Einstellung einer Zielkonzentration eines Wirkstoffes oder Medikamentes im Körper des Patienten ausgebildet ist, wobei die Bedieneinheit (24) ein Inkrementierungsbedienelement (30) zum Inkrementieren der Zielkonzentration und ein Dekrementierungsbedienelement (32) zum Dekrementieren der Zielkonzentration aufweist, wobei die Steuereinheit (12) ausgebildet ist, bei einer Betätigung des Inkrementierungsbedienelementes (30) die Zielkonzentration zu erhöhen und bei einer Betätigung des Dekrementierungsbedienelementes (32) die Zielkonzentration zu verringern, und/oder dass die Bedieneinheit (24) ein Ausleitungsbedienelement (36) zur Verringerung der Zielkonzentration auf einen vorgegebenen Wert, insbesondere für eine Narkoseausleitung, insbesondere auf null, aufweist, wobei die Steuereinheit (12) ausgebildet ist, bei einer Betätigung des Ausleitungsbedienelementes (36) eine der Zielkonzentration zugeordnete Flussrate der Infusionslösung, insbesondere graduell, insbesondere auf null, zu verringern.

## Beschreibung

Die Erfindung betrifft eine Infusionspumpe nach dem Oberbegriff von Anspruch 1.

Die Entwicklung in der modernen Medizin, insbesondere der Intensivmedizin, hat zu Infusionstherapien geführt, die den gezielten Einsatz und die exakte Dosierung hochwirksamer Medikamente erfordern. Im Rahmen dieser Therapien müssen je nach Krankheitsbild oftmals mehrere Medikamente verabreicht werden. Somit besteht ein Bedarf an modularen Infusionseinrichtungen, welche in der Lage sind, einfach mit mehreren Fluidpumpen/Infusionspumpen ausgerüstet zu werden. Hierfür ist es üblich, die Infusionspumpen als Verbund während des Einsatzes in oder an oder mittels einer Trage- oder Halteeinrichtung anzuordnen, um eine bestimmungsgemäße Bedienung sowie einen bestimmungsgemäßen Einsatz der Pumpen sicherzustellen.

Für die Target Conrolled Infusion (TCI) Anwendung ist vor allem eine Spritzenpumpe / ein Perfusor angedacht. Dieser hat eine höhere Genauigkeit bei der Dosierung. Ein Antriebskopf des Perfusors wird am Spritzenkolben einer eingelegten Spritze angeordnet. Der Antriebskopf wird mittels eines Gewindeantriebs, das von einem Schrittmotor angetrieben wird, in Richtung eines Spritzenzylinders bewegt. Dabei wird der Spritzenkolben relativ zum Spritzenzylinder verfahren und die Infusionslösung aus dem Spritzenzylinder verdrängt. Mit jedem Schritt des Schrittmotors wird somit eine Abgabe der Infusionslösung mit einem Medikament bzw. Wirkstoff bewirkt.

Eine Infusionslösung ist dabei insbesondere eine Flüssigkeit mit einem Wirkstoff bzw. Medikament, insbesondere umfassend Propofol, Remifentanil, Sufentanil, Fentanil, Alfentanil oder Dexmedetomidin.

Bei der Target Controlled Infusion (TCI) bzw. der zielwertgesteuerten Infusion wird die gewünschte Konzentration des Medikamentes bzw. Wirkstoffes im Patienten eingestellt. Dabei wird mit Hilfe wenigstens eines mathematischen Modells aus der gewünschten Zielkonzentration und weiteren Parametern bzw. Daten, welche beispielsweise das Medikament und den Patienten betreffen, eine entsprechende Flussrate berechnet. Die Pumpe wird dann entsprechend betrieben, um diese Flussrate zu realisieren. Die Steuereinheit der Infusionspumpe steuert die Pumpe aufgrund des Modells mit der Förderrate an, welche ein Ausgangswert des Modells ist. Das Modell bildet hierbei zumindest teilweise den Stoffwechsel des Patienten ab. Das Drei-Kompartment-Modell trägt als Grundlage zu TCI bei. Basis für dieses Modell bzw. Algorithmus sind validierte Studien. Für die Zulassung von TCI müssen somit keine weiteren Studien durchgeführt werden. Das TCI-Verfahren und das Drei-Kompartiment-Modell sind beispielsweise beschrieben in D. J. Eleveld, P. Colin, A. R. Absalom and M. M. R. F. Struys: Pharmacokinetic-pharmacodynamic model for propofol for broad application in anaesthesia and Sedation. British Journal of Anaesthesia, 120 (5): 942-959 (2018).

Aus der DE 20 2021 103 512 U1 ist eine Infusionspumpe mit verschwenkbarer Peristaltikpumpeneinheit und Touchdisplay bekannt.

Die DE 10 2017 111 299 A1 offenbart eine Infusionspumpe mit einem verschiedene Betriebszustände einnehmbaren Pumpmodul.

Nachteilig bei bekannten Pumpen für TCI sind die aufwändige Bedienung, Eingabe und Steuerung, insbesondere schlechte bzw. begrenzte Einstellungsmöglichkeiten und Zugänglichkeit.

Der Erfindung liegt daher die Aufgabe zugrunde, eine Infusionspumpe für TCI dahingehend zu verbessern, dass eine gezielte Einstellung der Zielkonzentration zuverlässig und sicher ermöglicht wird. Weiterhin soll ein entsprechendes Verfahren angegeben werden.

In Bezug auf die Infusionspumpe wird diese Aufgabe erfindungsgemäß gelöst mit einer Infusionspumpe mit den Merkmalen von Anspruch 1. Die Infusionspumpe umfasst dabei ein Gehäuse, eine Bedieneinheit, eine Antriebseinheit zur Förderung einer Infusionslösung und eine Steuereinheit, die für Target Controlled Infusion von Infusionslösung durch einen Patientenanschluss an einen Patienten zur Einstellung einer Zielkonzentration eines Wirkstoffes oder Medikamentes im Körper des Patienten ausgebildet ist, wobei die Bedieneinheit ein Inkrementierungsbedienelement zum Inkrementieren der Zielkonzentration und ein Dekrementierungsbedienelement zum Dekrementieren der Zielkonzentration aufweist, wobei die Steuereinheit ausgebildet ist, bei einer Betätigung des Inkrementierungsbedienelementes die Zielkonzentration zu erhöhen und bei einer Betätigung des Dekrementierungsbedienelementes die Zielkonzentration zu verringern, und/oder wobei die Bedieneinheit ein Ausleitungsbedienelement zur Verringerung der Zielkonzentration auf einen vorgegebenen Wert, insbesondere für eine Narkoseausleitung, insbesondere auf null, aufweist, wobei die Steuereinheit ausgebildet ist, bei einer Betätigung des Ausleitungsbedienelementes eine der Zielkonzentration zugeordnete Flussrate der Infusionslösung, insbesondere graduell, insbesondere auf null, zu verringern.

Vorteilhafte Ausgestaltungen der Erfindung sind Gegenstand der Unteransprüche.

Die Erfindung geht von der Überlegung aus, dass in modernen medizinischen Anwendungen bei TCI eine genaue Einstellung der Zielkonzentration notwendig ist, damit auf die jeweils vorliegende medizinische Situation des Patienten kurzfristig und zuverlässig reagiert werden kann und der Anwender bei der Arbeit entlastet wird.

Wie nunmehr erkannt wurde, lassen sich diese Anforderungen erfüllen, indem die Bedieneinheit zwei dedizierte Bedienelemente zum Inkrementieren bzw. Erhöhen und zum Dekrementieren bzw. Verringern der Zielkonzentration aufweist. Auf diese Weise ist eine Erhöhung oder Verringerung der Zielkonzentration direkt und jederzeit möglich. Eine umständliche Einstellung der Zielkonzentration, d.h. die Medikamenten- bzw. Wirkstoffkonzentration im Körper des Patienten, über eine Menüführung entfällt. Bevorzugt ist in der Infusionspumpe einstellbar, welche Konzentration eingestellt wird.

Die Steuereinheit stellt die Zielkonzentration im Körper des Patienten bevorzugt durch eine Einstellung der Flussrate ein, wobei die Flussrate aus der Zielkonzentration mit Hilfe des TCI-Modells und patientenspezifischer Parameter, beispielsweise Geschlecht, Gewicht, Alter, berechnet wird.

Auf dem Inkrementierungsbedienelement ist vorteilhafterweise ein Pfeil dargestellt, welcher bei der für den regulären Betrieb aufgestellten Infusionspumpe nach oben weist. Auf dem Dekrementierungsbedienelement ist vorteilhafterweise ein Pfeil dargestellt, welcher bei der für den regulären Betrieb aufgestellten Infusionspumpe nach unten weist. Die beiden Pfeile weisen somit in entgegengesetzte Richtungen.

Erfindungsgemäß weist die Bedieneinheit ein Ausleitungsbedienelement zur vollständigen Verringerung der Zielkonzentration bzw. Flussrate auf null, insbesondere graduell, auf, wobei die Steuereinheit ausgebildet ist, bei einer Betätigung des Ausleitungsbedienelementes die Zielkonzentration oder die Flussrate auf einen Wert reduziert wird, der insbesondere einer Zielkonzentration zur Ausleitung der Narkose zugeordnet ist, auf null zu verringern. Dies bedeutet, dass durch die Betätigung des Ausleitungselementes die Infusion beendet wird, im Falle einer Sedierung sinkt somit die Konzentration auf den Schwellenwert für die minimale Zielkonzentration ab, was typischerweise zu einem Aufwachen des Patienten führt.

Bei Betätigung des Ausleitungsbedienelementes wird dabei die Flussrate oder Zielkonzentration, insbesondere graduell, d.h. insbesondere schrittweise oder kontinuierlich, auf null reduziert. Die Verringerung der Zielkonzentration bzw. Flussrate erfolgt bevorzugt nicht abrupt, sondern mit einem negativen Gradienten. Insbesondere die Kombination der Inkrementierungs-/Dekrementierungsbedienelemente und des Ausleitungselementes ermöglicht eine sichere und kontrollierte Bedienung der Infusionspumpe. Das Ausleitungsbedienelement ist bevorzugt in einem unteren Bereich der Bedieneinheit angeordnet, so dass es gut für den Bediener eindeutig und leicht erkennbar ist. Vorteilhafterweise umfasst es eine textliche Kennzeichnung wie "Set to zero" oder ein Symbol.

Das Inkrementierungsbedienelement, das Dekrementierungsbedienelement und das Ausleitungsbedienelement sind Bedienelemente, welche unmittelbar zugänglich sind und insbesondere auf einer Frontklappe, insbesondere einem gemeinsamen Pumpendisplay, angeordnet sind. Vorteilhafterweise erfüllt jedes dieser Bedienelemente nur seine einzige Funktion und wird nicht für mehrere Funktionalitäten eingesetzt, wodurch die sichere Bedienung der Infusionspumpe verstärkt wird.

Die Inkrementierungsschrittweite und/oder die Dekrementierungsschrittweite hängt bevorzugt von in der Steuereinheit hinterlegten Daten, insbesondere von Daten aus einer Medikamentendatenbank und/oder patientenspezifischen Parametern, ab.

Die Inkrementierungsschrittweite und/oder die Dekrementierungsschrittweite kann sich an validierten pharmakokinetischen Modellen orientieren oder von diesen mit abhängen.

Auf diese Weise kann die Genauigkeit der Zielkonzentrationseinstellung an die konkrete Medikamenten- und/oder Patientensituation angepasst werden. Vorteilhafterweise sind Inkrementierungsschrittweite und Dekrementierungsschrittweite gleich groß und betragen bevorzugt 0,1 mcg/ml (Mikrogramm pro Milliliter).

Vorteilhafterweise ist in der Steuereinheit jeweils ein Schwellenwert für die maximale Zielkonzentration und/oder ein Schwellenwert für die minimale Zielkonzentration hinterlegt. Auf diese Weise kann sichergestellt werden, dass die Zielkonzentration und damit die Konzentration des Medikamentes im Patienten in Bereichen liegt, die kein gesundheitliches Risiko darstellen. Die Schwellenwerte sind jeweils vorzugsweise wirkstoff- bzw. medikamentenabhängig.

In einer vorteilhaften Ausführungsform ist die Bedieneinheit konfiguriert, eine Medikamentenauswahl einzustellen, wobei die Bedieneinheit konfiguriert ist, das Inkrementierungsbedienelement und/oder das Dekrementierungsbedienelement nur anzuzeigen und/oder aktiv zu schalten, wenn es sich für das ausgewählte Medikament eignet. Da sich nicht alle Medikamente zur Verwendung in TCI eignen, wird auf diese Weise sichergestellt, dass die Möglichkeit der Einstellung der Zielkonzentration nur erfolgen kann, wenn sich das entsprechende Medikament dafür eignet. Der Bediener der Infusionspumpe wird somit davor bewahrt, TCI bei nicht geeigneten Medikamenten anzuwenden.

Die Flussraten- bzw. Zielkonzentrationsänderung bei Betätigung des Ausleitungsbedienelementes hängt bevorzugt von in der Steuereinheit hinterlegten Daten, insbesondere patientenspezifischen Parametern, ab.

In einer bevorzugten Ausführungsform umfasst die Infusionspumpe eine Displayeinheit. Die Displayeinheit ist signaleingangsseitig mit der Steuereinheit verbunden und dient vorteilhafterweise dem Anzeigen von Daten bzw. Parametern, welche die TCI betreffen.

Vorteilhafterweise umfasst die Displayeinheit eine Zielkonzentrationsanzeige, sodass die Zielkonzentration direkt für den Bediener ablesbar ist. Die Zielkonzentrationsanzeige ist bevorzugt, insbesondere direkt, neben den Bedienelementen zur Inkrementierung bzw. Dekrementierung der Zielkonzentration angeordnet, wodurch dem Bediener die Bedeutung der beiden Elemente verdeutlicht wird und mögliche Fehlbedienungen verhindert werden.

In der Steuereinheit der Infusionspumpe sind vorteilhafterweise Informationen abgelegt, welche den Medikamentennamen und die Art und Weise, wie die Therapie zu administrieren ist. Die Infusionspumpe weist dazu vorteilhafterweise eine kabel- und/oder drahtlose Verbindungseinheit zur Verbindung mit einer Medikamentendatenbank auf.

Bevorzugt umfasst die Infusionspumpe bzw. ihre Displayeinheit ein Medikamentenanzeige, in der das aktuell verwendete Medikament angezeigt wird. Dadurch ist diese Information für den Bediener immer und direkt zugänglich. Auf der Displayeinheit sind weiterhin bevorzugt vorgesehen eine Anzeige der Konzentration des Medikamentes sowie eine Anzeige der bisher insgesamt dem Patienten während der Infusion verabreichten Medikamentenmenge. Weitere Daten, die auf der Displayeinheit angezeigt werden, sind vorteilhafterweise Flussrate, Medikament / Wirkstoff, Restlaufzeit, Konzentrationslevel, Wirkstoffkonzentration in der Infusionslösung, Betriebsmodus, Förderung aktiv, Gesamtwirkstoffmenge, TCI-Modell.

In einer vorteilhaften Ausgestaltung umfasst die Displayeinheit eine Förderratenanzeige / Konzentrationslevel-anzeige / Zielkonzentration. Die Anzeige zeigt zumindest eine Zielkonzentration an. Wenn mehrere Zielkonzentrationen angezeigt werden, werden diese bevorzugt mit unterschiedlichen Farben und/oder Bildsprache angezeigt. Dadurch wird eine höhere Informationsdichte im gleichen Bereich erzielt sowie eine klare Informationswiedergabe. Dadurch erfolgt eine Entlastung des Bedieners. Durch die klare Darstellung werden Bedienfehler reduziert; die Bedienung der Infusionspumpe ist weniger fehleranfällig, was zur Sicherheit des Patienten beiträgt.

Die Anzeige der jeweiligen Flussrate erfolgt bevorzugt als Funktion der Zeit in einer Kurve, insbesondere in einer zeitlich zyklischen Darstellung. Es erfolgt somit eine zyklische Ausgabe der Flussrate auf der Kommunikationsschnittstelle zum Nachzeichnen der Graphen. Dabei wird bevorzugt der Verlauf der Flussrate(n) in einem vorgegebenen Zeitintervall, beispielsweise 30 min, dargestellt. Dabei zeigt der Graph sowohl den Therapieverlauf unterschiedliche Zielkonzentrationen in der Vergangenheit als auch der prognostizierte Verlauf in die Zukunft, sobald die Therapie gestoppt wird.

Vorteilhafterweise ist die Displayeinheit als Touchdisplay ausgebildet, wobei die Bedieneinheit auf dem Touchdisplay angeordnet ist. Die Bedienelemente, d.h. Inkrementierungsbedienelement, Dekrementierungsbedienelement, Ausleitungsbedienelement, sind dabei Regionen auf dem Touchdisplay, welche auf eine Berührung reagieren und auf diese Weise betätigt werden.

In einer ersten bevorzugten Ausführungsform ist das Touchdisplay als resistives Touchdisplay ausgebildet, wodurch auch eine Bedienung mit gewöhnlichen Handschuhen möglich ist.

In einer weiteren bevorzugten Ausführungsform ist das Touchdisplay als kapazitives oder induktives Touchdisplay ausgebildet ist, wodurch eine Bedienung mit weniger Druck als bei einem resistiven Touchdisplay ermöglicht wird.

Die Antriebseinheit der Infusionspumpe kann ein Pumpenmodul aufweisen, wobei die Infusionspumpe einen Versorgungsanschluss für die Infusionslösung bzw. Flüssigkeit und einen Patientenanschluss zur Abgabe der Infusionslösung aufweist.

Die Infusionspumpe ist in einer vorteilhaften Ausbildung als Spritzenpumpe ausgebildet. Die Antriebseinheit weist dabei bevorzugt einen Schrittmotor auf zur Förderung von der Infusionslösung durch den Patientenanschluss. Bei der Betätigung des Inkrementierungsbedienelementes bzw. Dekrementierungsbedienelementes wird dann der Schrittmotor zur Erzielung der gewünschten Zielkonzentration angesteuert.

In Bezug auf das Verfahren wird die oben genannte Aufgabe gelöst durch ein Verfahren nach Anspruch 12. Dazu ist vorgesehen, dass mit Hilfe einer Infusionspumpe, die für Target Controlled Infusion von Infusionslösung durch einen Patientenanschluss an einen Patienten zur Einstellung einer Zielkonzentration eines Wirkstoffes oder Medikamentes im Körper des Patienten ausgebildet ist, Infusionslösung einem Patienten zugeführt wird, wobei eine Zielkonzentration mit Hilfe von auf einer Bedieneinheit der Infusionspumpe angeordneten Bedienelemente, insbesondere durch Erhöhen und Verringern eingestellt wird und/oder dass mit Hilfe eines von auf der Bedieneinheit der Infusionspumpe angeordneten Ausleitungsbedienelements die Zielkonzentration auf einen vorgegebenen Wert, insbesondere für eine Narkoseausleitung, insbesondere null, durch entsprechende Anpassung der Flussrate der Infusionslösung verringert wird.

Vorteilhafterweise wird die wenigstens eine Zielkonzentration auf einer Anzeige der Infusionspumpe überwacht, wobei bedarfsweise die Zielkonzentration mit Hilfe der Bedienelemente angepasst wird.

In einer bevorzugten Ausführungsform des Verfahrens wird die TCI mit Hilfe eines dafür vorgesehenen Bedienelementes auf der Bedieneinheit der Infusionspumpe ausgeleitet bzw. ausgeschlichen.

Die Vorteile der Erfindung liegen insbesondere darin, dass durch die Bedienelemente zum Erhöhen und Verringern bzw. Reduzieren/Erniedrigen der Zielkonzentration eine zuverlässige Medikamentierung des Patienten ermöglicht und gewährleistet wird. Mittels Bedienflächen auf der Bedieneinheit, insbesondere einem Touchdisplay, wird eine agile und akkurate Infusionspumpenbedienung im sogenannten Target Controlled Infusion (TCI) Modus erzielt. Eine one-touch- bzw. multi-touch-Anpassung der Zielkonzentration ist sehr einfach, unmittelbar und schnell durchführbar, was zu einer Entlastung des Bedieners für andere Aufgaben führt. Mit Hilfe des Ausleitungsbedienelements wird eine one-touch-Bedienung zur Ausleitung der Narkose statt einer aufwändigen Menu-Führung oder Einstellung ermöglicht.

Ein Ausführungsbeispiel der Erfindung wird anhand einer Zeichnung näher erläutert. Darin zeigen in stark schematisierter Darstellung:
- FIG. 1: eine Perspektivansicht einer Infusionspumpe gemäß eines bevorzugten Ausführungsbeispiels;
- FIG. 2: ein Touchdisplay mit einer Bedieneinheit einer Infusionspumpe in einer bevorzugten Ausführungsform, und
- FIG. 3: ein Ablaufdiagramm eines Verfahrens in einer bevorzugten Ausführungsform.

Gleiche Teile sind in allen Figuren mit denselben Bezugszeichen versehen.

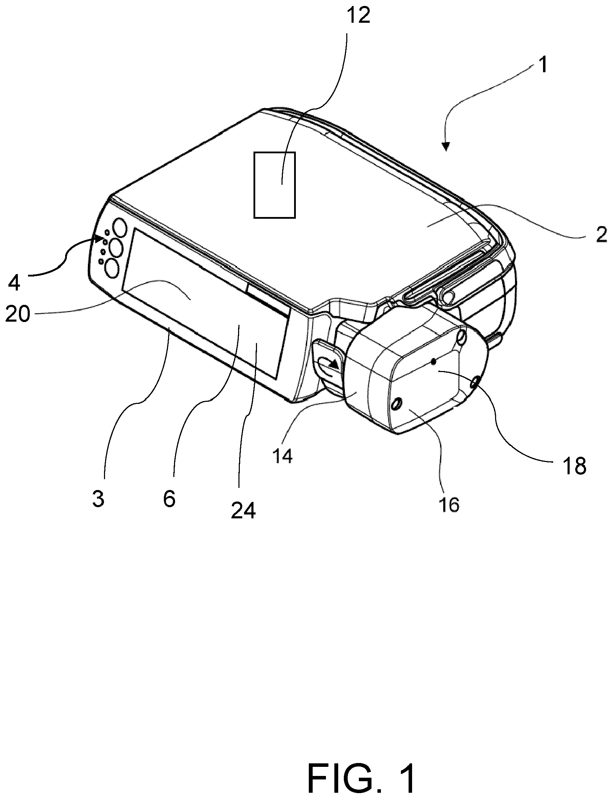

In Fig. 1 ist eine medizinische Infusionspumpe 1 gemäß eines bevorzugten Ausführungsbeispiel dargestellt, welche als Spritzenpumpe ausgebildet ist. Die Infusionspumpe 1 weist ein im Wesentlichen quaderförmiges Gehäuse 2 auf, an dessen Vorderseite eine Frontklappe 3 angelenkt ist. Auf einer Vorderseite der Frontklappe 3 sind mehrere Bedientasten 4 und eine Displayeinheit 20 angeordnet, welche als Touchdisplay 6 ausgebildet ist und eine Bedieneinheit 24 umfasst.

Die Infusionspumpe 1 weist eine im Gehäuse 2 schematisch angedeutete Steuereinheit 12 auf. Die Infusionspumpe 1 weist weiterhin eine Antriebseinheit 18 mit einem Antriebskopf 14 und einem Antriebskopfgehäuse 16 sowie einem Schrittmotor auf.

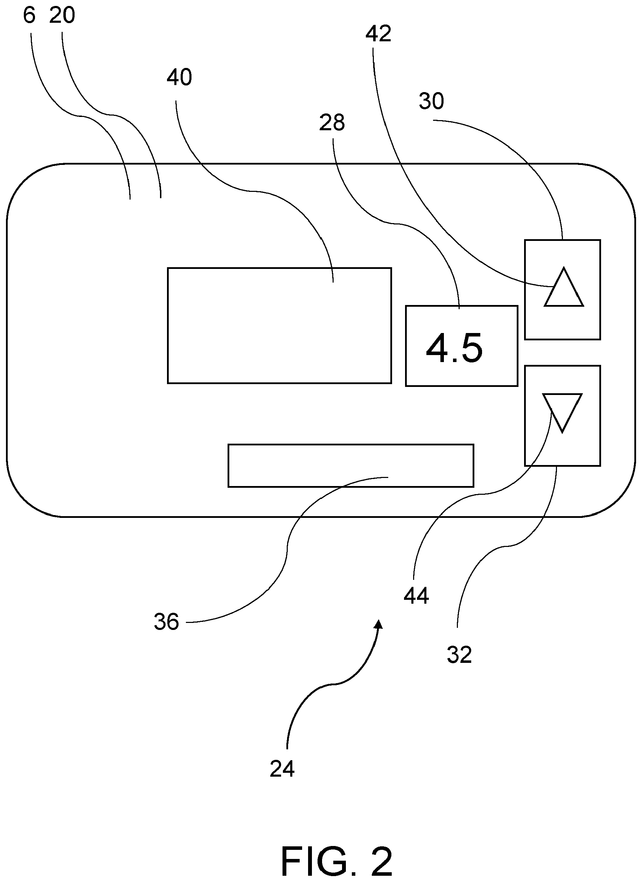

In FIG. 2 ist das Touchdisplay 6 der Infusionspumpe in einer bevorzugten Ausbildung schematisch dargestellt. Die Bedieneinheit 24 der Infusionspumpe 1 ist als eine Mehrzahl von Elementen auf dem Touchdisplay 6 ausgebildet.

Die Bedieneinheit 24 umfasst eine Zielkonzentrationsanzeige 28, welche die Zielkonzentration im Patienten anzeigt. Die Zielkonzentrationsanzeige 28 ist bevorzugt digital bzw. alphanumerisch ausgeführt. Sie zeigt die Zielkonzentration bevorzugt in der Einheit mcg/ml an. Die Bedieneinheit 24 weist weiterhin ein Inkrementierungsbedienelement 30 und ein Dekrementierungsbedienelement 32 auf. Die Bedieneinheit 24 weist weiterhin ein Ausleitungsbedienelement 36 auf. Die Bedienelemente 30, 32, 36 sind signaleingangsseitig mit der Steuereinheit 12 verbunden. Das Inkrementierungsbedienelement 30 weist einen als nach oben zeigendes Dreieck ausgebildeten Pfeil 42, das Dekrementierungsbedienelement 32 einen als nach unten zeigendes Dreieck ausgebildeten Pfeil 44 auf.

Die Steuereinheit 12 ist derart ausgebildet bzw. konfiguriert, dass sie bei einer Betätigung des Inkrementierungsbedienelementes 30 die Zielkonzentration erhöht, sofern die eingestellte Zielkonzentration nicht einen vorgegebenen maximalen Schwellenwert überschreitet. Die Steuereinheit 12 ist weiterhin derart ausgebildet bzw. konfiguriert, dass sie bei einer Betätigung des Dekrementierungsbedienelementes 32 die Zielkonzentration verringert, sofern die eingestellte Zielkonzentration nicht einen vorgegebenen minimalen Schwellenwert unterschreitet. Erhöhung und Verringerung erfolgt jeweils nach dem Target Controlled Infusion (TCI) Verfahren, welches in der Steuereinheit 12 software- und/oder hardwaremäßig implementiert ist.

Die Inkrementierungsschrittweite, in der bei Betätigung des Inkrementierungsbedienelementes 30 die Zielkonzentration erhöht wird und die Dekrementierungsschrittweite, in der bei Betätigung des Dekrementierungsbedienelementes 32 die Zielkonzentration verringert wird, sind in der Steuereinheit 12 hinterlegt.

Die Steuereinheit 12 ist dazu ausgebildet bzw. konfiguriert, bei einer Betätigung des Ausleitungsbedienelementes 36 die Zielkonzentration auf einen vorgegeben Wert, insbesondere auf null, zu verringern. Der vorgegebene Wert kann einem Wert für eine Narkoseausleitung entsprechen. Dies erfolgt bevorzugt nicht abrupt, sondern in der Form, dass die Flussrate stetig verringert wird, bis sie den vorgegeben Wert, beispielsweise null, erreicht.

Das Touchdisplay 6 weist eine Flussratenanzeige 40 auf, auf welcher wenigstens eine Flussrate dargestellt wird. Bei der Darstellung von mehreren Zielkonzentrationen werden diese mit unterschiedlichen Farben dargestellt, wodurch ihre schnelle Erkennbarkeit und Unterscheidung vereinfacht werden. Bei der Verwendung der Infusionspumpe zum Einleiten eines Sedierungsmedikamentes werden so die Sedierungsverläufe in Kurven graphisch dargestellt, welchen den prognostizierten Verlauf der Medikamenten- bzw. Wirkstoffkonzentration im Blut und/oder im Gehirn zeigen. Mit Hilfe dieser Kurven kann der Bediener beispielsweise erkennen, wie die durchgeführte Sedierung anflutet oder abflutet. Die Flussratenanzeige zeigt bevorzugt jeweils den prognostizierten Verlauf in einem vorgegeben Zeitintervall an. Das Zeitintervall kann beispielsweise 30 Minuten betragen, wobei jeweils die letzten 10 Minuten und die kommenden 20 Minuten dargestellt werden.

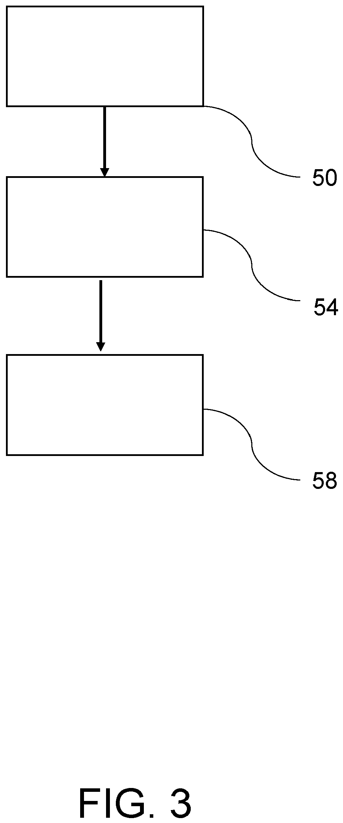

Ein Verfahren zur Durchführung einer Target Controlled Infusion bei einem Patienten, mit Hilfe einer Infusionspumpe 1 ist in FIG. 3 schematisch dargestellt. In einem Block 50 wird das Verfahren begonnen. Zu diesem Zeitpunkt ist der Patientenanschluss der Infusionspumpe durch einen Schlauch und eine Kanüle mit dem Blutkreislauflauf des Patienten bereits begonnen.

In einem Block 54 wird eine Zielkonzentration eines Medikamentes mit Hilfe von auf einer Bedieneinheit 24 der Infusionspumpe 1 angeordneten Bedienelemente 30, 32, insbesondere durch Erhöhen und Verringern bzw. Vermindern eingestellt. Bevorzugte Medikamente/Wirkstoffe sind Propofol, Remifentanil, Sufentanil, Fentanil, Alfentanil, Dexmedetomidin.

In einem Block 58 wird die wenigstens eine Zielkonzentration auf einer Anzeige 28 der Infusionspumpe überwacht, wobei bedarfsweise die Zielkonzentration mit Hilfe der Bedienelemente in Block 54 angepasst wird.

### Bezugszeichenliste

- 1: Infusionspumpe
- 2: Gehäuse
- 3: Frontklappe
- 4: Bedientasten
- 6: Touchdisplay
- 12: Steuereinheit
- 14: Antriebskopf
- 16: Antriebskopfgehäuse
- 18: Antriebseinheit
- 20: Displayeinheit
- 24: Bedieneinheit
- 28: Zielkonzentrationsanzeige
- 30: Inkrementierungsbedienelement
- 32: Dekrementierungsbedienelement
- 36: Ausleitungsbedienelement
- 40: Flussratenanzeige
- 42: Pfeil
- 44: Pfeil
- 50: Block
- 54: Block
- 58: Block

## Patentansprüche

1. Infusionspumpe (1) mit einem Gehäuse (2), einer Bedieneinheit (24), einer Antriebseinheit (18) zur Förderung einer Infusionslösung und einer Steuereinheit (12), die für Target Controlled Infusion von Infusionslösung durch einen Patientenanschluss an einen Patienten zur Einstellung einer Zielkonzentration eines Wirkstoffes oder Medikamentes im Körper des Patienten ausgebildet ist,
**dadurch gekennzeichnet, dass**
die Bedieneinheit (24) ein Inkrementierungsbedienelement (30) zum Inkrementieren der Zielkonzentration und ein Dekrementierungsbedienelement (32) zum Dekrementieren der Zielkonzentration aufweist, wobei die Steuereinheit (12) ausgebildet ist, bei einer Betätigung des Inkrementierungsbedienelementes (30) die Zielkonzentration zu erhöhen und bei einer Betätigung des Dekrementierungsbedienelementes (32) die Zielkonzentration zu verringern, und/oder dass die Bedieneinheit (24) ein Ausleitungsbedienelement (36) zur Verringerung der Zielkonzentration auf einen vorgegebenen Wert, insbesondere für eine Narkoseausleitung, insbesondere auf null, aufweist, wobei die Steuereinheit (12) ausgebildet ist, bei einer Betätigung des Ausleitungsbedienelementes (36) eine der Zielkonzentration zugeordnete Flussrate der Infusionslösung, insbesondere graduell, insbesondere auf null, zu verringern.

2. Infusionspumpe (1) nach Anspruch 1, wobei die Inkrementierungsschrittweite und/oder die Dekrementierungsschrittweite von in der Steuereinheit (12) hinterlegten Daten, insbesondere von Daten aus einer Medikamentendatenbank und/oder patientenspezifischen Parametern, abhängen.

3. Infusionspumpe (1) nach Anspruch 1 oder 2, wobei in der Steuereinheit (12) ein Schwellenwert für die maximale Zielkonzentration und/oder ein Schwellenwert für die minimale Zielkonzentration hinterlegt sind.

4. Infusionspumpe (1) nach einem der Ansprüche 1 bis 3, wobei die Bedieneinheit (24) konfiguriert ist, eine Medikamentenauswahl einzustellen, und wobei die Bedieneinheit (24) konfiguriert ist, das Inkrementierungsbedienelement (30) und/oder das Dekrementierungsbedienelement (32) nur anzuzeigen und/oder aktiv zu schalten, wenn das ausgewählte Medikament zu einer vorgegeben Medikamentengruppe für Target Controlled Infusion gehört.

5. Infusionspumpe (1) nach einem der vorherigen Ansprüche, umfassend eine signalausgangsseitig mit der Steuereinheit (12) verbundene Displayeinheit (20).

6. Infusionspumpe (1) nach Anspruch 5 wobei die Displayeinheit (20) eine Zielkonzentrationsanzeige umfasst.

7. Infusionspumpe (1) nach Anspruch 5 oder 6, wobei die Displayeinheit (20) eine Flussratenanzeige (40) umfasst.

8. Infusionspumpe (1) nach einem der Ansprüche 5 bis 7, wobei die Displayeinheit (20) als Touchdisplay (6) ausgebildet ist, und wobei die Bedieneinheit (24) auf dem Touchdisplay (6) angeordnet ist.

9. Infusionspumpe (1) nach Anspruch 8, wobei das Touchdisplay (6) als resistives Touchdisplay (6) ausgebildet ist.

10. Infusionspumpe (1) nach Anspruch 8, wobei das Touchdisplay (6) als kapazitives oder induktives Touchdisplay (6) ausgebildet ist.

11. Infusionspumpe (1) nach einem der vorherigen Ansprüche, welche als Spritzenpumpe ausgebildet ist.

12. Verfahren zur Durchführung einer Target Controlled Infusion bei einem Patienten, wobei mit Hilfe einer Infusionspumpe (1), die für Target Controlled Infusion von Infusionslösung durch einen Patientenanschluss an einen Patienten zur Einstellung einer Zielkonzentration eines Wirkstoffes oder Medikamentes im Körper des Patienten ausgebildet ist, Infusionslösung einem Patienten zugeführt wird,
**dadurch gekennzeichnet, dass**
eine Zielkonzentration mit Hilfe von auf einer Bedieneinheit (24) der Infusionspumpe (1) angeordneten Bedienelemente (30, 32), insbesondere durch Erhöhen und Verringern eingestellt wird und/oder dass mit Hilfe eines von auf der Bedieneinheit (24) der Infusionspumpe (1) angeordneten Ausleitungsbedienelements (36) die Zielkonzentration auf einen vorgegeben Wert, insbesondere für eine Narkoseausleitung, insbesondere null, durch entsprechende Anpassung der Flussrate der Infusionslösung verringert wird.

13. Verfahren nach Anspruch 12, wobei die wenigstens eine Zielkonzentration auf einer Anzeige der Infusionspumpe (1) überwacht wird, und wobei bedarfsweise die Zielkonzentration mit Hilfe der Bedienelemente (30, 32) angepasst wird.
